# EUROPEAN PATENT APPLICATION

(11) **EP 2 820 999 A1**
(43) Date of publication of application: **07.01.2015**
(21) Application number: 13755079.4
(22) Date of filing: 26.02.2013
(51) Int. Cl.: A61B 1/00, G02B 23/24

(54) **ENDOSCOPE**

(30) Priority: 27.02.2012 JP 2012040406
(71) Applicant: Olympus Medical Systems Corp., Tokyo 151-0072 (JP)
(72) Inventor: OMOTO, Keijiro, Hachioji-shi Tokyo 192-8512 (JP)
(74) Representative: Schicker, Silvia
(86) International application number: PCT/JP2013/054997
(87) International publication number: WO 2013/129416

(57) **Abstract**

An endoscope (1) comprises an insertion unit (2) having a bending portion (9) on a distal end side thereof, an endoscope main body (4) provided on a proximal end side of the insertion unit (2), a left-and-right direction bending mechanism (13) coupled with the bending portion (9), a left-and-right direction bending drive unit (23) which generates drive force to bend the bending portion (9) in a left-and-right direction, a drive force transmission mechanism (22) which transmits the drive force to the left-and-right direction bending mechanism (13) and a cord portion (5) which extends from the endoscope main body (4) and supplies electrical power from the outside to the left-and-right direction bending drive unit (23). The left-and-right direction bending drive unit (23) is provided on the endoscope main body (4) and arranged along an extending direction of the cord portion (5).

## Description

### Technical Field

The present invention relates to an endoscope having a bending portion which bends in upper and lower directions and left and right directions.

### Background Art

In the medical field or the industrial field, an endoscope having an elongated insertion unit that is inserted into a body cavity or a duct, which is a so-called flexible scope, is extensively used. In endoscopy or surgical operation under endoscope, such a medical endoscope is inserted into a body cavity along a bent shape of, for example, a small intestine or a large intestine while a bending operation unit is operated to bend a bending portion on a distal end side of an insertion unit in upper and lower directions and left and right directions. Further, an image of a desired observation region is obtained by an observation optical system on a distal end surface of the insertion unit, and observation, diagnosis, capturing, and others are carried out based on this image.

For example, Patent Literature 1 discloses an endoscope in which a first bending portion and a second portion on a distal end side of an insertion unit, two first bending operation units that bend the first bending portion in upper and lower directions and left and right directions, and a second bending operation unit that bends the second bending portion are provided. In this endoscope, for example, the first bending operation unit and the second bending operation unit are revolvaly provided around a first axis and a second axis respectively, and the first axis is arranged to be orthogonal to the second axis.

### Patent Literature

Patent Literature 1: Jpn. Pat. Appln. KOKAI Publication No. 2004-283618

### Summary of Invention

The endoscope is gripped by one hand of an operator, and then the bending operation unit for the upper and lower directions and the bending operation unit for the left and right directions are operated. If the operator is skillful at operating the endoscope, he/she can perform operations for bending the bending portions in the upper and lower directions and the left and right directions with use of the bending operation units in one hand.

However, it is difficult for an operator who has small hands or a beginner who lacks experience of operating the endoscope to operate each bending operation unit in one hand only, and he/she must operate while additionally using the other hand as support. For example, in case of inserting the endoscope into a body cavity which has continuous bent parts and a complicated shape like a large intestine, an operator needs to hold the insertion unit in the other hand in order to assure a position of the insertion unit. Therefore, it is not practical for an operator who has small hands or a beginner to operate each bending operation unit in one hand, and the operation is very difficult.

Thus, to facilitate the operation of each bending operation unit by a one-handed operation for the operator who has small hands or the beginner, the bending operation in the left and right directions is motorized, and each bending operation unit is arranged at a position where the one-handed operation can be easily performed. Even in case of such a type of electrical endoscope, it is preferable to maintain existing endoscope operability so that the endoscope can be stably gripped and operated without shaking it.

It is an object of the present invention to provide an endoscope that can maintain existing operability even if the endoscope is of a type that a bending portion on a distal end side of an insertion unit can be electrically bent in left and right directions.

In an embodiment of the present invention, an endoscope comprising: an insertion unit having a bending portion on a distal end side thereof; an endoscope main body provided on a proximal end side of the insertion unit; a left-and-right direction bending mechanism coupled with the bending portion; a left-and-right direction bending drive unit which generates drive force to bend the bending portion in a left-and-right direction; a drive force transmission mechanism which transmits the drive force to the left-and-right direction bending mechanism; and a cord portion which extends from the endoscope main body and supplies electrical power from the outside to the left-and-right direction bending drive unit, wherein the left-and-right direction bending drive unit is provided on the endoscope main body and arranged along an extending direction of the cord unit.

According to the present invention, there can be provided an endoscope that can maintain existing operability even if the endoscope is of a type that a bending portion on a distal end side of an insertion unit can be electrically bent in left and right directions.

### Brief Description of Drawings

FIG. 1 is a view showing an endoscopic system including an endoscope according to a first embodiment of the present invention;
FIG. 2 is a view schematically showing a bending portion, a flexible tube portion, and other components related to a bending operation in upper and lower directions of the bending portion in an endoscope main body;
FIG. 3 is a view schematically showing the bending portion, the flexible tube portion, and other components related to a bending operation in left and right direction of the bending portion in the endoscope main body;
FIG. 4 is a view schematically showing a transmission configuration of a drive mechanism for an RL bending operation in an endoscope main body;
FIG. 5 is a view showing the endoscope main body gripped by an operator's hand and the inside thereof;
FIG. 6 is a view showing a transmission configuration of a drive unit in the endoscope main body;
FIG. 7 is a side view showing an aspect of the endoscope main body;
FIG. 8 is a side view showing an aspect of the endoscope main body;
FIG. 9 is a side view showing another aspect of the endoscope main body;
FIG. 10 is a perspective view showing a switch box depicted in FIG. 9; and
FIG. 11 is a view showing a transmission configuration of a drive mechanism for an RL bending operation and a UD bending operation in an endoscope main body of an endoscope according to a second embodiment of the present invention.

### Brief Description of Embodiments

### [First Embodiment]

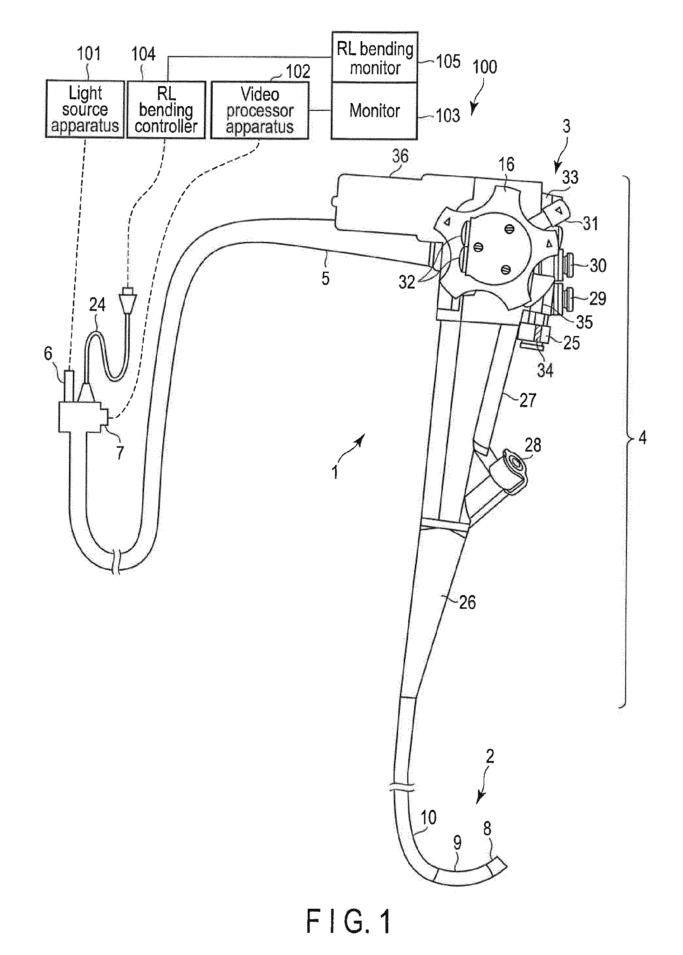

A first embodiment of the present invention will now be described with reference to FIG. 1 to FIG. 10.

FIG. 1 is a view showing an endoscopic system 100 including an endoscope 1 according to the first embodiment of the present invention. The endoscope 1 roughly has an elongated insertion unit 2 on an distal end side of the endoscope, an endoscope main body 4 which is coupled with a proximal end side of the insertion unit 2 and includes an operation unit 3, and a universal cord 5 which is extended from the endoscope main body 4 and includes internal constituent members such as a light guide, an electrical cable, and others which are accommodated in the insertion unit 2 and the endoscope main body 4.

An endoscopic system 100 has the endoscope 1, a light source apparatus 101 which leads illumination light to the endoscope 1, a video processor apparatus 102 which converts an electrical signal obtained by the endoscope 1 into an image signal and outputs it, and a monitor 103 which displays an image based on the image signal output from the video processor apparatus 102. A light source connector 6 and an electrical connector 7 are provided to a distal end side of the universal cord 5 of the endoscope 1, the light source connector 6 is connected to the light source apparatus 101, and the electrical connector 7 is connected to the video processor apparatus 102 through a cable.

The insertion unit 2 is an elongated tubular portion on the distal end side of the endoscope which is inserted into a body cavity or the like. The insertion unit 2 has a distal end portion 8 that is provided at the outermost end of the insertion unit 2, a bending portion 9 provided on a proximal end side of the distal end portion 8, and a long flexible tube portion 10 provided on the proximal end side of the bending portion 9.

The distal end portion 8 is a hard portion whose outer peripheral surface is made of a hard material such as stainless and which is covered with a distal end portion cover made of a synthetic resin. Although not shown, in the distal end portion 8, an observation optical system including an objective lens arranged on a distal end surface, a solid-state image sensing element such as a CCD that forms an optical image obtained from the observation optical system and converts it into an electrical signal, an illumination optical system including an illumination lens arranged on the distal end surface, a light guide which leads illumination light to the illumination optical system, an air supply/water supply channel for lens cleaning, a forceps channel for forceps insertion, and others are provided.

The light guide is extended from the distal end portion 8 to the light source connector 6 on the distal end side of the universal cord 5 through the bending portion 9, the flexible tube portion 10, and the endoscope main body 4. The light guide transmits the illumination light output from the light source apparatus 101 connected to the light source connector 6 to the illumination optical system of the distal end portion 8, and the illumination light is emitted from the illumination lens on the distal end surface. The air supply/water supply channel is also extended from the distal end portion 8 to a non-illustrated water supply tube on the distal end side of the universal cord 5 through the bending portion 9, the flexible tube portion 10, and the endoscope main body 4.

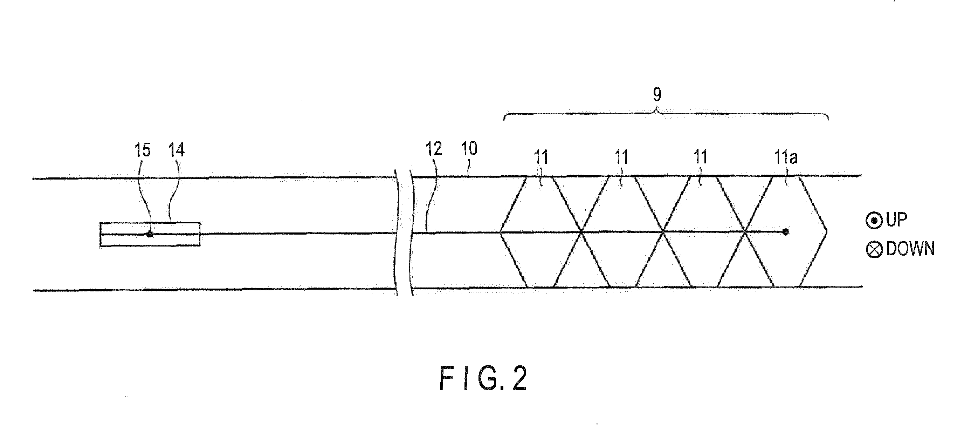

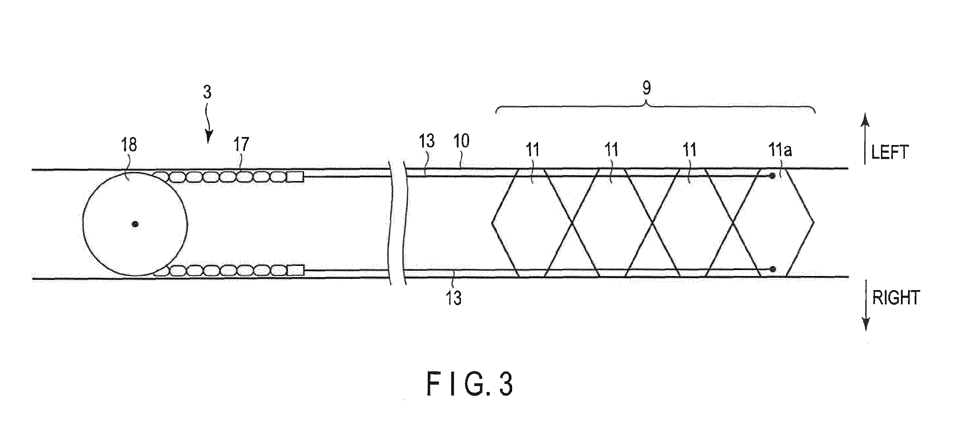

Each of FIG. 2 and FIG. 3 is a view schematically showing a configuration concerning the bending portion 9, the flexible tube portion 10, and other components related to a bending operation of the bending portion 9 in the endoscope main body 4. Bending pieces 11 are arranged in the bending portion 9 along a longitudinal axis direction of the insertion unit 2. These bending pieces 11 are revolvaly coupled with each other. These bending pieces 11 are covered with a bending blade obtained by plaiting thin wires or the like into a cylindrical form, and an upper side of the bending blade is covered with bending rubber made of, for example, fluororubber. The flexible tube portion 10 is an elongated soft tube which is made of fluororesin or the like and has flexibility.

In the bending portion 9, as shown in FIG. 2, a distal end of a UD (up/down) bending operation wire 12 as an up-and-down direction bending mechanism is coupled with the outermost distal end bending piece 11a at a position corresponding to an up-and-down direction of the bending portion 9. Further, as shown in FIG. 3, a distal end of an RL (right/left) bending operation wire 13 as a left-and-right direction bending mechanism is coupled with the outermost distal end bending piece 11a at a position corresponding to a left-and-right direction of the bending portion 9.

As shown in FIG. 2, the UD bending operation wire 12 is extended from the outermost distal end bending piece 11a of the bending portion 9 into the endoscope main body 4 through the flexible tube portion 10, and a proximal end of this wire is wound around a rotary drum 14. A rotary shaft of a UD bending operation knob 16 as an up-and-down direction bending operation input unit which operates bending (an angle) of the bending portion 9 in the up-and-down direction is attached to a rotary shaft 15 of the rotary drum 14. Therefore, when the UD bending operation knob 16 is rotated, the bending portion 9 bends in the upper direction or the lower direction.

As shown in FIG. 3, the RL bending operation wire 13 is extended from the outermost distal end bending piece 11a of the bending portion 9 into the endoscope main body 4 through the flexible tube portion 10, and a proximal end of this wire is coupled with a chain 17 through a connection member. The chain 17 is wound around a sprocket 18, and the sprocket 18 is coupled with an RL bending drive unit 19.

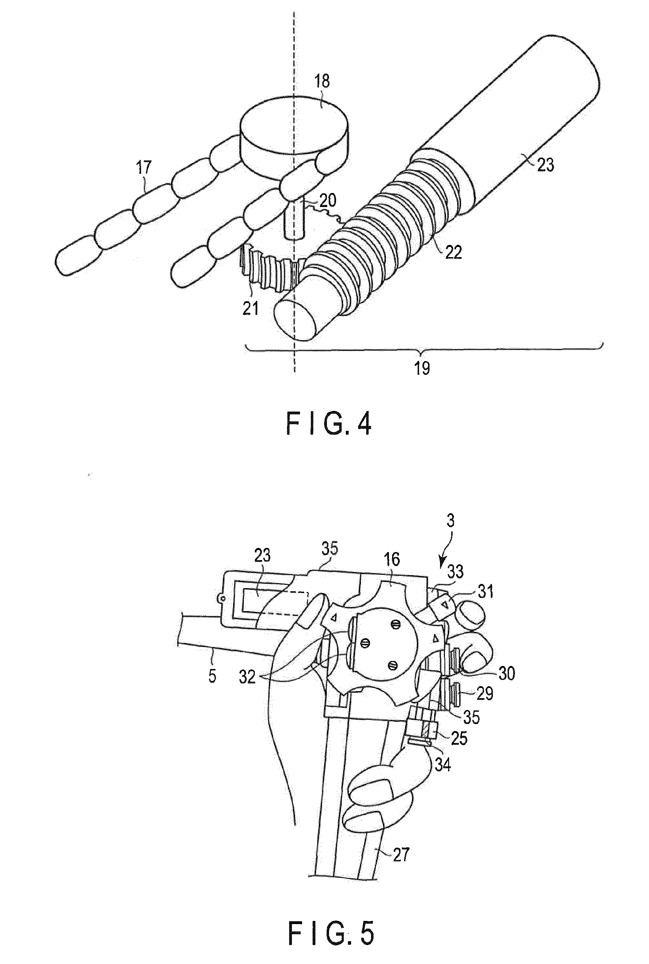

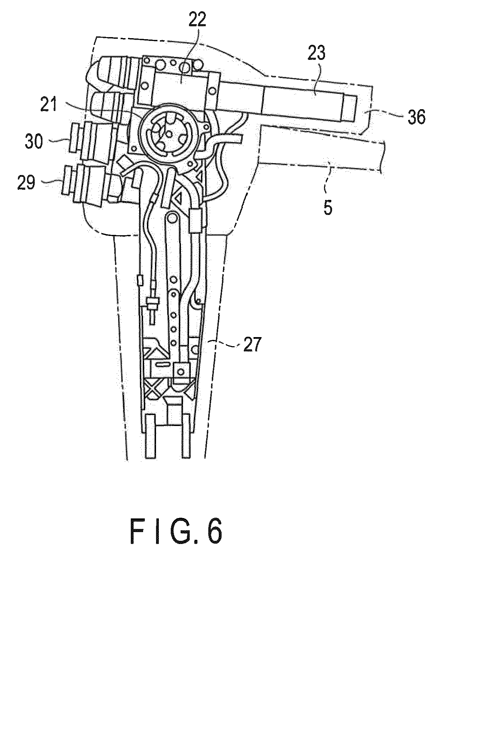

FIG. 4 is a view schematically showing a transmission configuration of the RL bending drive unit 19 which is a drive mechanism for the RL bending operation in the endoscope main body 4. FIG. 5 is a view showing the endoscope main body 4 including a grip portion 27 gripped by an operator's hand and the operation unit 3 and the inside thereof. FIG. 6 is a view showing a transmission configuration of the RL bending drive unit 19 in the endoscope main body 4.

The RL bending drive unit 19 has: a drive power transmission mechanism including a worm wheel 21 coaxially connected with the sprocket 18 through a shaft 20 and a worm gear 22 that meshes with the worm wheel 21; and an RL bending drive motor 23 as a left-and-right direction bending drive unit coupled with the worm gear 22.

As shown in FIG. 1, the RL bending drive motor 23 is connected to an RL bending controller 104 from a distal end of the electrical cable in the universal cord 5 through a motor drive power supply cable 24. Thus, electrical power is supplied to the RL bending drive motor 23 from the outside through the universal cord 5. Furthermore, an RL bending operator 25 as a left-and-right direction bending operation input unit is also connected to an RL bending controller 104 through the universal cord 5.

When a bending operation signal indicative of a left-and-right direction bending operation input to the RL bending operator 25 is output to the RL bending controller 104, the RL bending controller 104 drives the RL bending drive motor 23 in accordance with this bending operation signal. Then, the RL bending drive motor 23 generates drive force for bending the bending portion 9 in the left-and-right direction, and the RL bending operation wire 13 is moved through the drive force transmission mechanism. In this manner, when the RL bending operator 25 is operated (rotated), namely, when an instruction for bending the bending portion 9 in the left-and-right direction is input to the RL bending operator 25, the bending portion 9 is electrically bent in the left direction or the right direction.

The RL bending controller 104 is also connected to an RL bending monitor 105 which is provided together with the monitor 103. As a result, an amount of bending in the left-and-right direction is displayed in the RL bending monitor 105. In FIG. 1, although the RL bending monitor 105 is separated from the monitor 103, an amount of bending in the left-and-right direction may be displayed in the monitor 103.

It should be noted that, in regard to operations of the bending portion 9 at the time of inserting the insertion unit 2 into a meandering body cavity, the up-and-down direction and left-and-right direction bending operations are not actually equivalent to each other, a main operation is the up-and-down direction bending operation, and the left-and-right direction bending operation is supplementarily used at the time of observation and the like. In this embodiment, therefore, the up-and-down direction bending operation is performed by a manual operation mechanism, and the left-and-right direction bending operation is motorized.

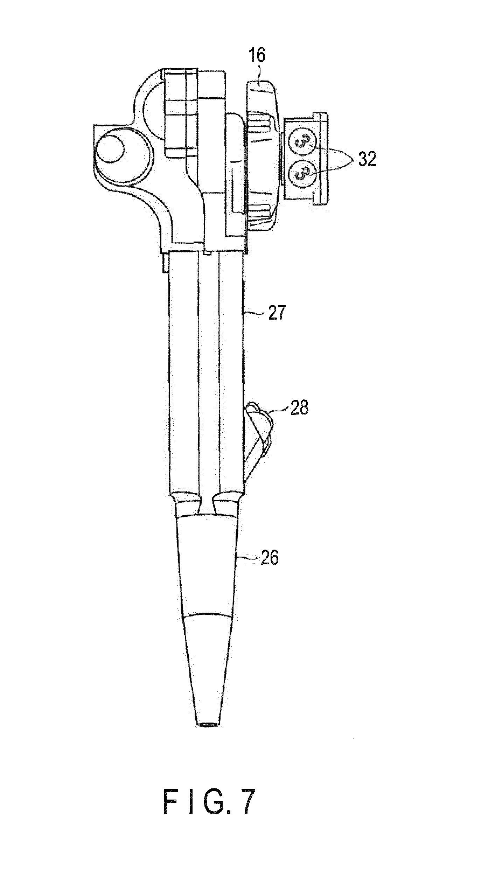

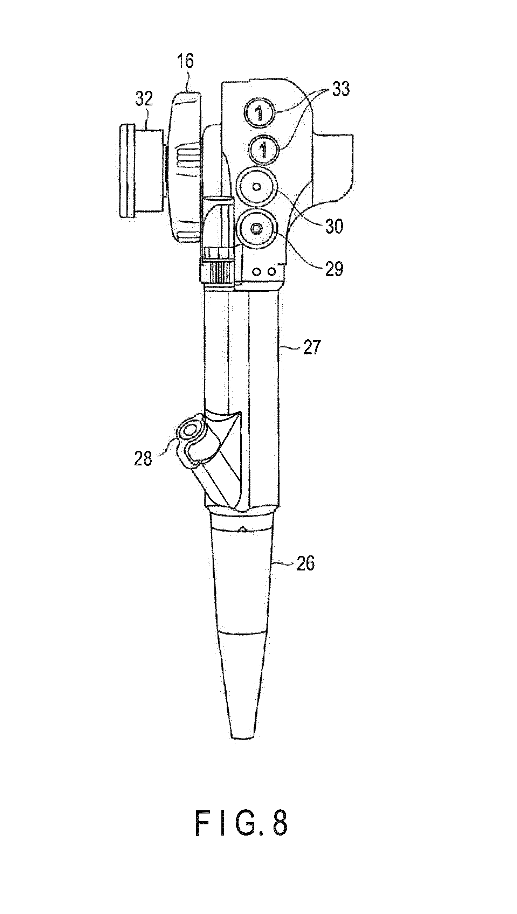

Each of FIG. 7 and FIG. 8 is a side view showing an aspect of the endoscope main body 4.

A support portion 26 that supports the proximal end of the flexible tube portion 10 is provided on the distal end side of the endoscope main body 4. The distal end of the support portion 26 has a taper shape that narrows toward the proximal end of the flexible tube portion 10 of the insertion unit 2. A grip portion 27 that is gripped by an operator as shown in FIG. 5 is provided on the proximal end side of the support portion 26.

A forceps insertion opening 28 is provided to the grip portion 27. The forceps insertion opening 28 communicates with the above-mentioned forceps channel formed in the insertion unit 2. A surgical instrument such as an ultrasonic probe or biopsy forceps is inserted into the forceps insertion opening 28 to resect, stanch, or sample a lesioned part in a body cavity. It should be noted that the forceps insertion opening 28 and the forceps channel can be also used as a suction opening and a suction channel as will be described later.

The operation unit 3 that performs various kinds of operations for the endoscope 1 including the bending operation of the bending portion 9 is provided on the proximal end side of the grip portion 27. The operation unit 3 has the UD bending operation knob 16 configured to bend the bending portion 9 in the up-and-down direction, the above-mentioned RL bending operator 25 configured to bend the bending portion 9 in the left-and-right direction, an air supply/water supply button 29, a suction button 30, a UD bending operation fixing lever 31, and function switches 32 and 33.

The UD bending operation knob 16 is rotatably provided on a first shaft portion protruding from one side surface (a first position) of the operation unit 3 of the microscope main body 4, and it is rotated with a thumb of one hand of an operator who is gripping the grip portion 27 in one hand (a left hand) being placed thereon. As a result, the UD bending operation wire 12 is operated, and the bending portion 9 is operated in the upper direction or the lower direction. That is, when an operation of bending the bending portion 9 in the up-and-down direction is input to the UD bending operation knob 16, the bending portion 9 is mechanically bent in the upper direction or the lower direction. The UD bending operation fixing lever 31 is a brake that fixes the bending portion 9 at a desired angle.

The air supply/water supply button 29 and the suction button 30 are arranged on the other side surface of the operation unit 3 (a side facing the side from which the universal cord 5 is extended), and they are pressed by a middle finger of one hand of an operator who is gripping the grip portion 27 in one hand as shown in FIG. 5. A small hole is formed at, for example, the center of the air supply/water supply button 29, air is supplied through the air supply/water supply channel when the operator closes this small hole with his/her finger, and water is supplied when the button is pressed. The suction button 30 is configured to suck and remove water droplets or mucus adhering to the distal end portion 8 through the forceps channel when this button is pressed.

The function switches 32 are arranged on an upper surface of the UD bending operation knob 16. Important functions such as capturing an image of an observed region or magnification of an image are assigned to the function switches 32 by setting the video processor apparatus 102. Furthermore, other function switches 33 are likewise arranged on the side surface where the air supply/water supply button 29 and the suction button 30 are provided. Functions of the function switches 33 are, for example, switching of photometry, stoppage of an image, and others.

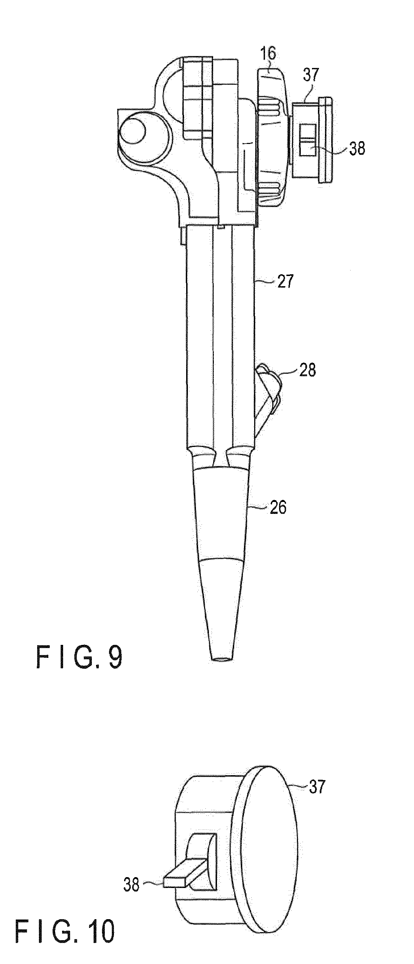

Each of FIG. 9 and FIG. 10 is a side view showing another aspect of the endoscope main body 4.

In another aspect, a switch box 37 is arranged on the upper surface of the UD bending operation knob 16, and a function lever 38 is provided on the switch box 37. The function lever 38 is a seesaw switch that changes over on/off of its function when it is pivoted. A function such as capturing an image of an observed region is likewise assigned to the function lever 38.

As shown in FIG. 1 and FIG. 5, the RL bending operator 25 is rotatably provided on a second shaft portion, which is protruded from a second position placed on the grip portion 27 side apart from the first position where the UD bending operation knob 16 is provided, to be substantially along the longitudinal axis direction of the endoscope main body 4. In other words, the RL bending operator 25 is arranged with a rotary shaft substantially parallel to the longitudinal axis direction of the grip portion 27 below the air supply/water supply button 29 and the suction button 30. The RL bending operator 25 is also rotated by a middle finger of one hand of an operator who is holding the grip portion 27 in one hand.

A marker 34 is provided on an outer surface of the RL bending operator 25. Furthermore, a corresponding marker 35 is also provided on the operation unit 3 above the RL bending operator 25. These markers 34 and 35 are lines or marks which are engraved or printed in or on the RL bending operator 25 and the operation unit 3, they can indicate an amount of rotation of the RL bending operator 25. The marker 34 is provided at, for example, a neutral point of the RL bending operator 25, and a neutral position can be provided by matching this point to the marker 35 in the longitudinal axis direction. It should be noted that the marker 35 of the operation unit 3 is supplementary, and it does not have to be provided.

In this embodiment, as shown in FIG. 5, the RL bending drive motor 23 as the left-and-right direction bending drive unit configured to bend the bending portion 9 in the left-and-right direction and a motor accommodating portion 36 accommodating the motor are arranged on the endoscope main body 4 along the universal cord 5 as an external lead cord potion for extending each internal constituent member in the endoscope main body 4 along a substantially orthogonal direction from the endoscope main body 4 extending in the longitudinal axis direction. Here, the internal constituent member means the insertion unit 2 and a component running through the inside of the endoscope main body 4 from the insertion unit 2, for example, an endoscope main body light guide, an electrical cable, a water supply pipe, or the like.

For example, in case of arranging the RL bending operation unit at a position to which even an operator with small hands or a beginner who is not skillful at operating the endoscope can place a finger of his/her hand gripping the grip portion of the endoscope main body, it is desirable to arrange an operator of the RL bending operation unit having a desired size in desired arrangement in the operation unit. This can be achieved by adopting an electrical bending mechanism for bending in the left-and-right direction alone. Here, a position of center of gravity of the operation unit depending on the arrangement of the drive unit in the electrical bending mechanism greatly affects operability.

For example, in case of arranging the drive unit of the electrical bending mechanism in the grip portion, a space to accommodate the drive unit must be provided in the grip portion, and the grip portion is elongated for the space. Therefore, when an operator operates the operation unit, the operation can become unstable.

Further, in case of arranging the drive unit on the proximal end side of the endoscope apart from a position where the operation unit is gripped, i.e., the universal cord side, considering a structure of a hand, how to grip the endoscope, how to operate the endoscope, and others, arranging each component so that the center of gravity of the operation unit can become closer to a base position of a thumb of one hand holding the grip portion can facilitate fitting of the operation unit in a palm at the time of operating the endoscope, thereby helping the grip. Since the operation unit in an existing endoscope is also configured so that the position of center of gravity center can be placed near the base position of the thumb of one hand holding the grip portion, it can be considered that this configuration enables maintaining the operational feeling of the existing endoscope.

Thus, in this embodiment, the RL bending drive motor 23 as the drive unit and its accommodating portion 36 are arranged in the same direction as the universal cord 5 so that it can be along the universal cord 5, whereby center of gravity of the operation unit can be placed near the base position of the thumb. As a result, even the endoscope which is of a type that the bending portion is electrically bent in the left-and-right direction, the operability of the existing endoscope can be maintained without elongating the grip portion, thereby enabling the stable operation. Further, the arrangement of the drive portion along the universal cord does not obstruct the operation.

Furthermore, the bending portion can be electrically bent in the left-and-right direction by the RL bending drive motor 23 and the RL bending operator 25 for the electrical operation is arranged at a position different from the position of the RL bending operation knob (the upper surface of the UD bending operation knob 16) of the regular endoscope, i.e., the lateral surface side that can be easily operated by using one hand holding the grip portion, whereby the function switches 32 configured to execute various functions of the endoscope can be arranged on this lateral surface side that can be easily operated by using one hand.

In the bending operation of the bending portion in the conventional endoscope, function switches are arranged at the positions of the function switches 33 alone. In case of pressing each function switch in such arrangement, it may not be possibly operated with use of only one hand gripping the grip portion. According to this embodiment, since the bending operation in the left-and-right direction is electrically performed, each function switch 32 having a particularly important (frequently used) function, for example, capturing can be arranged on the upper surface of the UD bending operation knob 16. As a result, like the operation for bending in the up-and-down direction, an operator can press the function switch 32 with a thumb of one hand gripping the grip portion 27, thereby improving the operability.

Moreover, since the function switch 32 is arranged near the UD bending operation knob 16, an operator can capture an observed region or perform magnified observation while maintaining an accurate position in the bending operation, and the operation can be carried out without losing sight of a position of the observed region.

As described above, according to this embodiment, it is possible to provide the endoscope that enables the electrical bending operation in the left-and-right direction while maintaining the bending operation in the up-and-down direction of the existing endoscope irrespective of a difference in usage experience or a size of a hand.

Additionally, in this embodiment, the worm gear is adopted for the drive force transmission mechanism. By adopting the worm gear, a speed can be greatly reduced by single deceleration, and the drive force transmission mechanism can be miniaturized.

Meanwhile, in the motorized RL bending operation, an operator operates the RL bending operator 25 and bends the bending portion 9 in the left-and-right direction while confirming a picture of an observed region in the monitor 103. At this time, a bent state is perceived while confirming an output from the RL bending controller 104 having the cable 24 from the universal cord 5 connected thereto in the RL bending monitor 105 or the like as means for confirming an amount of bending or a bending direction (a bent state).

However, nothing may be displayed in the RL bending monitor 105 during a procedure with some causes. In such a case, an operator cannot perceive a bent state at this moment, and hence the operation cannot be continued. In particular, in case of raising operation sensitivity by finely adjusting bending, since rotation of the RL bending operator 25 becomes multiple rotation, the operator cannot confirm an amount of bending in the left-and-right direction even more.

To cope with such a situation, in this embodiment, the markers 34 and 35 are provided to the RL bending operator 25 corresponding to the operation side that instructs to bend in the left-and-right direction and the operation unit 3, respectively, whereby an amount of bending can be double checked with use of the RL bending monitor 105 and the RL bending operator 25. As a result, even if the RL bending monitor 105 cannot display anything due to a failure or the like, an operator can visually confirm an amount of bending. Thus, the bending operation can be safely continued.

Additionally, at the time of inserting the insertion unit into a bent body cavity, for example, a large intestine, if an operator feels strangeness in regard to an operation performed by himself/herself and movement of an angle in display of the RL bending monitor 105, confirming the markers that are close to the operator's hand allows continuing the procedure while giving certainty in the operation.

### [Second Embodiment]

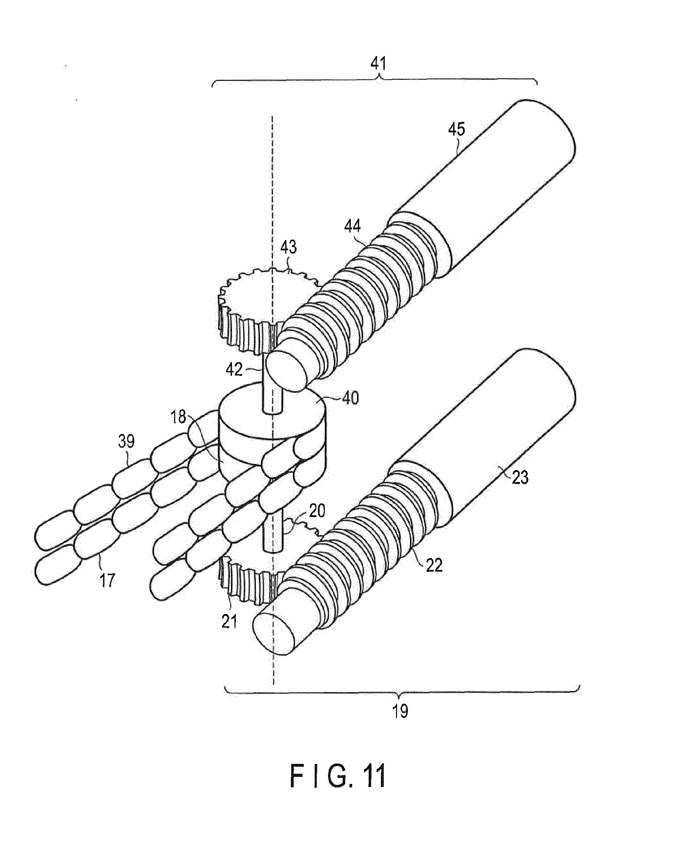

A second embodiment will now be described hereinafter with reference to FIG. 11. In the following description, like reference numbers denote the same components as those in the first embodiment, and a description thereof will be omitted.

In the first embodiment, the UD bending operation knob 16 configured to bend the bending operation 9 in the up-and-down direction is manually operated, and the bending in the left-and-right direction is motorized by the bending drive unit 19, but both the bending operations in the up-and-down direction and the left-and-right direction are motorized in the second embodiment. Further, both an RL bending drive motor 23 of an RL bending drive unit 19 and a UD bending drive motor 45 of a UD bending drive unit 41 are provided on an endoscope main body 4, and they are arranged along a universal cord 5 extending to the outside from a proximal end of the endoscope main body 4 which extends in the longitudinal axis direction.

FIG. 11 is a view schematically showing a transmission mechanism of the RL bending drive unit 19 and the RD bending drive unit 41 as drive mechanisms configured to perform an RL bending operation and a UD bending operation in the endoscope main body 4 according to the second embodiment. A configuration of the RL bending drive unit 19 is the same as that in the first embodiment.

In this embodiment, like the RL bending operation wire 13 shown in FIG. 3 in the first embodiment, a UD bending operation wire 12 extends from the outermost distal end bending piece 11a of a bending portion 9 into the endoscope main body 4 through a flexible tube portion 10, and a proximal end of this wire is coupled with a chain 39. The chain 39 is wound around a sprocket 40, and the sprocket 40 is coupled with the UD bending drive unit 41. The UD bending drive unit 41 has: a drive force transmission mechanism including a worm wheel 43 coaxially connected with a sprocket 40 through a shaft 42 and a worm gear 44 that meshes with the worm wheel 43; and a UD bending drive motor 45 as an up-and-down direction bending drive unit coupled with the worm gear 44.

Like the RL bending drive motor 23, the UD bending drive motor 45 is connected to a UD bending controller from a distal end of an electrical cable in the universal cord 5 through a motor drive power supply cable. Therefore, electrical power is supplied to the UD bending drive motor 45 from the outside through the universal cord 5. The UD bending controller may be separated from or may be integrated with an RL ben controller 104. Furthermore, a UD bending operation knob 16 is also connected to the UD bending controller through the universal cord 5.

When a bending signal indicative of an up-and-down direction bending operation input to the UD bending operation knob 16 is output to the UD bending controller, the UD bending controller drives the UD bending drive motor 45 in accordance with this bending operation signal. Moreover, the UD bending drive motor 45 generates drive force for bending the bending portion 9 in the up-and-down direction, thereby moving the UD bending operation wire 12 through the drive force transmission mechanism. When the UD bending operation knob 16 is operated (rotated) in this manner, the bending portion 9 is electrically bent in the upper direction or the lower direction.

The UD bending controller is also connected to a monitor 103 and a UD bending monitor provided together with an RL bending monitor 105. As a result, an amount of bending in the up-and-down direction is displayed in the UD bending monitor.

In this embodiment, like the RL bending drive motor 23 and the motor accommodating portion 36 shown in FIG. 5 in the first embodiment, the RL bending drive motor 23 as a left-and-right direction bending drive unit configured to bend the bending portion 9 in the left-and-right direction and a motor accommodating portion 36 accommodating this motor as well as the UD bending drive motor 45 as an up-and-down direction bending drive unit configured to bend the bending portion 9 in the up-and-down direction and a motor accommodating portion accommodating this motor are arranged along the universal cord 5 extending in a substantially orthogonal direction from the endoscope main body 4 extending in the longitudinal axis direction.

According to the second embodiment, when the RL bending drive motor 23 and the UD bending drive motor 45 as drive units are arranged in the same direction as the universal cord 5 to be along the universal cord 5 so that a gravity center of an operation unit can be equivalent to that of an existing endoscope, even the endoscope in which the bending operations of the bending portion in the up-and-down direction and the left-and-right direction are motorized can maintain operability of the existing endoscope.

Although the embodiments have been explained in this specification, the present invention is not restricted to the foregoing embodiments, it is obvious for persons skilled in the art that various modifications and changes can be made without departing from the scope of the present invention.

## Claims

1. An endoscope (1) **characterized by** comprising:
an insertion unit (2) having a bending portion (9) on a distal end side thereof;
an endoscope main body (4) provided on a proximal end side of the insertion unit (2);
a left-and-right direction bending mechanism (13) coupled with the bending portion (9);
a left-and-right direction bending drive unit (23) which generates drive force to bend the bending portion (9) in a left-and-right direction;
a drive force transmission mechanism (22) which transmits the drive force to the left-and-right direction bending mechanism (13); and
a cord portion (5) which extends from the endoscope main body (4) and supplies electrical power from the outside to the left-and-right direction bending drive unit (23),
wherein the left-and-right direction bending drive unit (23) is provided on the endoscope main body (4) and arranged along an extending direction of the cord portion (5).

2. The endoscope according to claim 1, **characterized in that**
the endoscope main body (4) has an operation unit (3) which operates the bending portion (9),
the operation unit (3) comprises:
an up-and-down direction bending operation input unit (16) which is provided to a first shaft portion (15), which is protruded from a first position of the operation unit (3), to allow its revolving motion and to which an operation input for bending the bending portion (9) in the left-and-right direction is input; and
a left-and-right direction bending operation input unit (25) which is provided to a second shaft portion, which is protruded from a second position placed on the grip portion side apart from the first position of the operation unit (3) to be substantially along a longitudinal axis direction of the endoscope main body (4), to allow its revolving motion and to which an operation input for bending the bending portion (9) in the left-and-right direction is input, and
the up-and-down direction bending operation input unit (16) is a rotary knob which is configured to perform a manual bending operation.

3. The endoscope according to claim 2, **characterized in that**
at least one function switch (32, 33) is provided on an upper surface of the up-and-down direction bending operation input unit (16).

4. The endoscope according to claim 1, **characterized in that**
the drive force transmission mechanism (22) comprises a worm gear.

5. The endoscope according to claim 1 **characterized in that**, further comprising:
an up-and-down direction bending mechanism (12) coupled with the bending portion (9);
an up-and-down direction bending drive unit (41) which generates drive force to bend the bending portion (9) in an up-and-down direction; and
a drive force transmission mechanism (45) which transmits the drive force to the up-and-down direction bending mechanism (12),
wherein the cord portion (5) supplies electrical power from the outside to the up-and-down direction bending drive unit (41), and
the up-and-down direction bending drive unit (41) is provided on the endoscope main body (4) and arranged along the extending direction of the cord portion (5).
